# EUROPEAN PATENT APPLICATION

(11) **EP 4 589 319 A1**
(43) Date of publication of application: **23.07.2025**
(21) Application number: 24152562.5
(22) Date of filing: 18.01.2024
(51) Int. Cl.: G01R 33/28, A61F 11/08, A61F 11/14, G01R 33/54, A61B 5/055, G01R 33/385, A61B 6/10, G01R 33/48, G06V 20/52, A61N 5/10

(54) **DEVICE, SYSTEM AND METHOD FOR PROTECTION OF A SUBJECT FROM NOISE DURING A MEDICAL PROCEDURE**

(71) Applicant: Koninklijke Philips N.V., 5656 AG Eindhoven (NL)
(72) Inventor: FORTHMANN, Peter, Eindhoven (NL); BOERNERT, Peter Ulrich, Eindhoven (NL)
(74) Representative: Philips Intellectual Property & Standards

(57) **Abstract**

The present invention relates to a device, system and method for protection of a subject from noise during a medical procedure. The device comprises an input unit (21) configured to obtain image data of the subject acquired while entering or within a medical apparatus just before and/or at the beginning of and/or during a medical procedure using the medical apparatus (30), a processing unit (22) configured to detect from the obtained image data if the subject is wearing ear protection, and an output unit (23) configured to output, if it is detected that the subject does not wear ear protection, a notification that the subject does not wear ear protection and/or a control signal configured to prevent the medical apparatus from starting the medical procedure or to modify the medical procedure.

## Description

### FIELD OF THE INVENTION

The present invention relates to a device, system and method for protection of a subject, e.g. a patient or, generally, a person, from noise during a medical procedure.

### BACKGROUND OF THE INVENTION

Most magnetic resonance (MR) examinations are so loud that patients are required to wear earmuffs for hearing protection. There is a trend towards silent scanning using either some methodological approaches, like zero echo time (ZTE) imaging or MR imaging (MRI) with very down-scaled gradients. Another approach is to use adapted and future magnet and gradient technology to get MR scans quiet enough for patients to enter the scanner without any hearing protection.

Thus, it is conceivable that entire MRI patient examinations may run soon in silent mode for special patient sub-populations (e.g., infants) or for answering dedicated clinical questions (e.g., basic simple brain exams), making the need for complete ear protection obsolete. This would not only improve patient comfort, but would also simplify clinical workflow, and would ease the engagement of medical staff from other modalities (like X-ray) who are not used to any kind of ear protection.

However, there may be situations where patients do not wear ear protection (also called hearing protection, such as earmuffs or headphones or ear plugs) although they should and situations where patients do not wear ear protection although they it is not required.

### SUMMARY OF THE INVENTION

It is an object of the present invention to ensure that a subject unintentionally does not suffer from loud noise produced by a medical procedure that the subject is undergoing.

In a first aspect of the present invention a device for protection of a subject from noise during a medical procedure is presented, the device comprising:
- an input unit configured to obtain image data of the subject acquired while entering or within a medical apparatus just before and/or at the beginning of and/or during a medical procedure using the medical apparatus;
- a processing unit configured to detect from the obtained image data if the subject is wearing ear protection; and
- an output unit configured to output, if it is detected that the subject does not wear ear protection, a notification that the subject does not wear ear protection and/or a control signal configured to prevent the medical apparatus from starting the medical procedure or to modify the medical procedure.

In a further aspect of the present invention a system for protection of a subject from noise during a medical procedure is presented, the system comprising:
- an image acquisition unit configured to acquire image data of the subject while entering or within a medical apparatus just before and/or at the beginning of and/or during a medical procedure using the medical apparatus; and
- a device as disclosed herein for protection of a subject from noise during a medical procedure based on the acquired image data.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device (herein also called protection device), in particular as defined in the dependent claims and as disclosed herein.

The present invention is based on the finding that problems may arise, if patients are wrongly told that they can leave the hearing protection off although they are going to be subjected to a loud medical procedure, such as a loud MR scan, or the operator accidentally selects a wrong protocol for the medical procedure, such as a wrong MR imaging protocol, that happens to be loud. Situations where a patient is not wearing hearing protection and a loud scan is selected are avoided according to the present invention by detecting if the patient is wearing hearing protection when he/she just enters the medical apparatus or is just placed within or on the medical apparatus or even when the medical procedure has just started. This detection can be made from images taken by an image acquisition unit, such as a camera, that monitors the area of the entrance and/or within or on the medical apparatus. Such a camera may thus be arranged in the examination room or at the top or within the medical apparatus, e.g. at the entrance or within a bore of an MRI apparatus.

In case the subject does not wear hearing protection, the operator or a control apparatus for controlling the medical procedure will be informed so that the medical procedure can be stopped or modified to avoid any discomfort or even damage of the patient cause by loud noise generated during the medical procedure.

While the present invention is mainly used in the field of MR exams, it can be used in the context of other medical procedures that produce loud noise when they are executed, such as some radio-therapeutical treatments or MR-Linac exams, metabolic imaging using MR-PET or other imaging modalities that are exposing the patient to some uncomfortable level of acoustic noise.

In an embodiment, the processing unit is configured to perform image processing of the obtained image data to detect if the subject is wearing ear protection. For instance, the head of a person can be detected, and it can then be detected if the person wears ear protection. Optionally, it may even be detected if the person is entering the medical apparatus and/or represents the patient to avoid that the detection is erroneously made for a medical staff person.

In another embodiment, the processing unit is configured to detect if the subject is wearing earmuffs, headphones or ear plugs, in particular by recognizing a pattern or form of earmuffs, headphones or ear plugs in one or more images of the obtained image data. Conventional image processing tools, such as pattern recognition, or a trained computer system or algorithm, such as a trained neural network or other artificial intelligence system may be used for this detection.

In another embodiment, the processing unit is configured to perform the detection and/or the output unit is configured to perform the output of the notification and/or control signal only if the medical apparatus is expected to generate noise above a noise threshold when performing the medical procedure and/or if the medical procedure is modified during its operation so that the medical apparatus is expected to generate noise above the noise threshold. Thus, only if really needed, the processing regarding the detection and/or output is carried out, which saves processing capacity. If a medical procedure generates noise above a noise threshold may be known from the scheduled operation itself or from an input by the operator. For instance, the expected noise level of a medical procedure, e.g., a certain MR protocol, may be taken from a database.

The output unit may be configured to output, if it is detected that the subject does wear ear protection and the medical apparatus is expected to generate noise below a noise threshold when performing the medical procedure, a notification that the subject may remove the ear protection. This provides improved patient comfort since it is avoided that a patient wears ear protection although not needed.

The noise threshold may be predetermined or settable by a user, in particular individual for the subject or the type of subject. For instance, for all patients the same (fixed) noise threshold may be used. In another embodiment, the noise threshold may be individual for each patient or group of patients. For instance, for children a lower noise threshold may be applied compared to adults).

As briefly mentioned above, the input unit may be configured to obtain one or more images from a camera that is configured to monitor the medical apparatus, in particular the area of examination and/or the entrance to the area of examination. The camera may be configured to transmit the acquired images to a central control station that controls the medical apparatus and that can stop, start or modify medical procedure if necessary.

The medical apparatus may preferably be a medical imaging apparatus, in particular a magnetic resonance imaging, MRI, apparatus, configured to acquire image data of the subject in a medical imaging procedure, and the input unit is configured to obtain image data from the medical imaging apparatus. However, the present invention may be applied in the context of other medical apparatuses and other medical procedures as well.

In an embodiment the output unit is configured to output a control signal configured to control the medical imaging apparatus to perform an initial image acquisition to acquire image data enabling the detection if the subject is wearing ear protection. Thus, instead of a separate camera, the medical imaging apparatus is used to acquire an initial image data set from which it can be detected if the patient wears ear protection or not.

For instance, as provided in an embodiment, the output unit may be configured to output a control signal configured to control the medical imaging apparatus to perform a silent image acquisition generating noise below a noise threshold, in particular to perform a zero echo time (ZTE) scan if the medical imaging apparatus is an MRI apparatus.
The proposed system comprises an image acquisition unit and a device as disclosed herein. As described above, the image acquisition unit may comprise a camera configured to monitor the medical apparatus, in particular the area of examination and/or the entrance to the area of examination, and/or a medical imaging apparatus, in particular a magnetic resonance imaging, MRI, apparatus, configured to acquire image data of the subject in a medical imaging procedure, wherein the medical apparatus may be configured to perform a medical procedure such as an MRI procedure.

### BRIEF DESCRIPTION OF THE DRAWINGS

These and other aspects of the invention will be apparent from and elucidated with reference to the embodiment(s) described hereinafter. In the following drawings
Fig. 1 shows a diagram of a first embodiment of a system according to the present invention.
Fig. 2 shows a schematic diagram of an embodiment of a device according to the present invention.
Fig. 3 shows a diagram of a second embodiment of a system according to the present invention.
Fig. 4 shows a schematic diagram of an embodiment of a method according to the present invention.

### DETAILED DESCRIPTION OF EMBODIMENTS

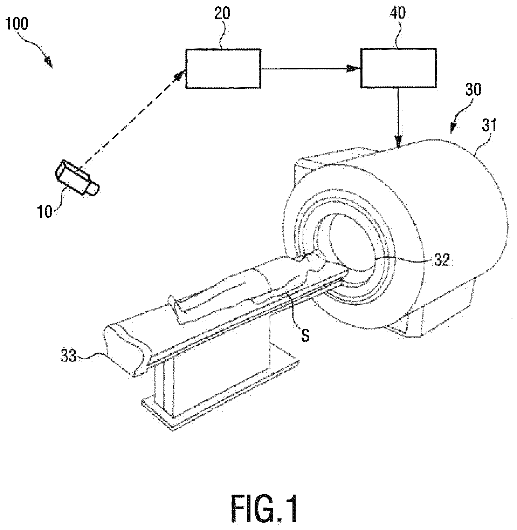

Fig. 1 shows a diagram of a first embodiment of a system 100 for protection of a subject S (e.g. a patient) from noise during a medical procedure according to the present invention. It comprises an image acquisition unit 10 and a device 20 (herein also called protection device) for protection of a subject S from noise during a medical procedure.

The image acquisition unit 10 is configured to acquire image data of the subject S while entering or within a medical apparatus 30 just before and/or at the beginning of and/or during a medical procedure using the medical apparatus 30. In this embodiment, the image acquisition unit 10 comprises a camera that monitors the area at and around the entrance, in this embodiment the bore 32 of the MRI apparatus 30 representing the medical apparatus.

The protection device 20 is configured to protect the subject S from noise during the medical procedure based on the image data acquired by the image acquisition unit 10. Details of the protection device 20 will be described below. The image acquisition unit 10 is, wirelessly or in a wired manner connected to the protection device 20.

The medical apparatus 30 in this embodiment is an MRI apparatus that comprises a magnetic unit 31 that defines a bore 32 into which the subject S can be moved by means of a patient table 33. The medical procedure is an MRI examination carried out by the MRI apparatus 30 that generally produces loud noise during the examination so that the subject S generally must wear ear protection, such as earmuffs, headphones or ear plugs.

The protection device 20 may be connected or integrated into a control apparatus 40, e.g. a workstation or computer, that controls the operation of the medical apparatus 30 and may hence use the information obtained from the protection device 20 to control the operation, e.g., start, stop or modify an examination. For instance, an interlock mechanism may be provided that prevents starting a loud protocol in case of a negative ear protection test or missing ear protection.

In addition or alternatively, the protection device 20 (and/or the control apparatus 40) may issue a notification or warning or recommendation if the subject should but does not wear ear protection. For instance, a corresponding notification may be issued, e.g., on a screen, for information of the operator or other staff so that they can provide the patient with the required ear protection.

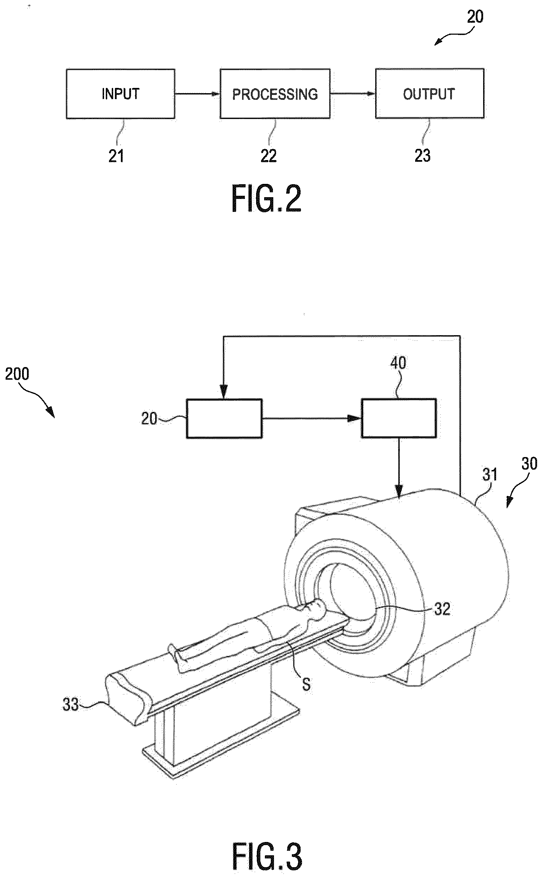

Fig. 2 shows a schematic diagram of an embodiment of a device 20 according to the present invention. The device 20 comprises an input unit 21, a processing unit 22 and an output unit 23.

The input unit 21 is configured to obtain image data of the subject S acquired while entering or within the medical apparatus 30 just before and/or at the beginning of and/or during a medical procedure using the medical apparatus 30. The input unit 21 may be directly coupled or connected to the image acquisition unit 10 or may obtain (i.e. retrieve or receive) these signals from a storage, buffer, network, or bus, etc. The input unit 21 may thus e.g. be a (wired or wireless) communication interface or data interface, such as a Bluetooth interface, Wi-Fi interface, LAN interface, HDMI interface, direct cable connection, or any other suitable interface allowing data transfer to the device 20.

The processing unit 22 is configured to detect from the obtained image data if the subject is wearing ear protection. The processing unit 22 may be any kind of means configured to process the image data and determine the desired information there from. It may be implemented in software and/or hardware, e.g. as a programmed processor or computer or app on a user device such as a smartphone, smartwatch, tablet, laptop, PC, workstation, etc.

The output unit 23 is configured to output, if it is detected that the subject does not wear ear protection, a notification that the subject does not wear ear protection and/or a control signal configured to prevent the medical apparatus from starting the medical procedure or to modify the medical procedure. The output unit 23 may generally be any interface that provides the determined notification and/or control signal, e.g. transmits it to control apparatus 40 or a user device, such as the operator's and/or other staff person's smartphone, computer, tablet, etc. It may thus generally be any (wired or wireless) communication or data interface.

Generally, the device 20 may be implemented by respective units or circuitry, e.g. a processor, processing circuitry, a computer, dedicated hardware, etc., that carries out the functions of the device. Alternatively, a common unit or circuitry, e.g. a common processor or computer, may implement the various functions of the device, or separate units or elements may be used that together represent the circuitry. In an exemplary implementation, a programmed processor may represent the device 20, which may execute a computer program that may be stored in a memory that is accessed by the processor.

Camera systems, as used in the first embodiment shown in Fig. 1, in MR settings are becoming more and more common. Using such a camera system makes it easy to record a patient and detect if the patient is wearing earmuffs or other ear protection systems or not. In case the patient is not wearing them, and a loud protocol is selected, maybe due to the need that became obvious during the scanning session or others, the operator will be notified and/or the control apparatus will automatically act so that the situation can be rectified, leading to a higher patient comfort and preventing any damage of the ears or any uncomfortable feelings during the examination.

In another embodiment, this approach can be applied the other way around. For instance, if a silent MR protocol is chosen, and the patient wears earmuffs, the operator may be notified that the patient could be told to take them off, again leading to increased patient comfort

Fig. 3 shows a diagram of a second embodiment of a system 200 according to the present invention. Compared to the system 100 shown in Fig. 1, no separate camera (10 in Fig. 1) is provided, but the medical apparatus 30 provides the function of the image acquisition unit. In particular, since the medical apparatus 30 is a medical imaging apparatus that is configured to acquire image data of the subject in a medical imaging procedure, it can be controlled, e.g. by the output unit 23 of the protection device 20 or by the control apparatus 40 to perform an initial image acquisition to acquire image data enabling the detection if the subject is wearing ear protection. Thus, before the actual medical procedure, i.e., the schedule imaging scan, is performed, a quick image acquisition is performed when the patient S is already at the right position within the bore 32 by the MRI apparatus 30. In this way, image data of the patient can be acquired, in particular of the head region in the area of the ears, which are sufficient to detect if the patient is wearing ear protection or not. The MRI apparatus 30 may e.g. be controlled to perform a silent image acquisition generating noise below a noise threshold, in particular to perform a zero echo time (ZTE) scan that does not harm the patient and is not as loud as a "normal" MRI scan.

In this embodiment the same functionality can be realized as in the first embodiment using the MRI apparatus as an ear protection check system. During the travel to scan period, moving the patient into the MRI apparatus, or after positioning the patient in the MRI bore, a silent MRI scan can be triggered to identify whether there are earmuffs in space. For example, in a brain examination a quick ZTE scan can be performed. This is an almost silent 3D scan that is sensitive to fast T2-relaxing components and runs in presence of an almost contact field gradient with neglectable gradient orientation change during the scan, reducing the sound pressure level dramatically. The plastics in the earmuffs, although being solid, can be visualized at the given TEs (e.g. 0.07ms). In this way, the presence or absence of ear protection can be detected via the MRI, triggering the above-mentioned actions.

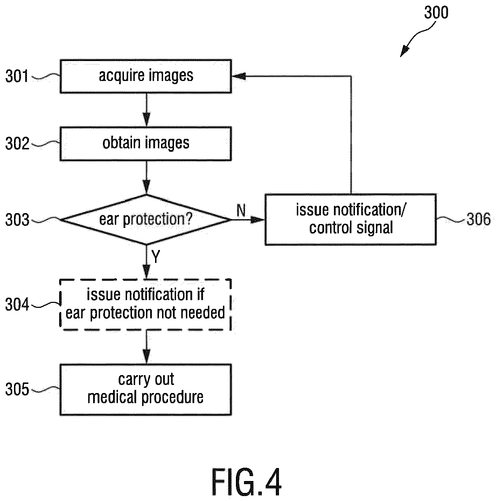

Fig. 4 shows a schematic diagram of an embodiment of a method 300 according to the present invention for protection of a subject from noise during a medical procedure. In a first step 301, image data are acquired by the image acquisition unit, such as a camera 10 or a medical imaging apparatus 30, while the subject is entering or within a medical apparatus just before and/or at the beginning of and/or during a medical procedure using the medical apparatus. In a second step 302, the image data are obtained by the protection device 20, e.g., they are transmitted from the image acquisition unit or retrieved from the image acquisition unit.

In a third step 303 it is detected from the obtained image data if the subject is wearing ear protection or not. If it is detected that the subject is wearing ear protection the medical procedure can be carried out as planned (step 305). As an optional intermediary step, if it is known or detected that there is no need to wear ear protection for the scheduled medical procedure, a notification may be issued (step 304) that the subject can take off the ear protection for the scheduled medical procedure.

If it is detected that the subject is not wearing ear protection although required for the scheduled medical procedure, a notification that the subject does not wear ear protection and/or a control signal to prevent the medical apparatus from starting the medical procedure or to modify the medical procedure is issued in step 306. Thereafter, the method may continue with step 301 to acquire and evaluate new image data to make sure that the subject really put on ear protection before the medical procedure is started.

Steps 302-306 of the method 300 may be carried out by the protection device 20, wherein the main steps of the method are carried out by the processing unit 22 of the protection device 20. The method may e.g. be implemented as computer program running on a computer or processor.

In summary, by the present invention an efficient and easily realizable solution is presented to protect a patient from discomfort or harm due to too loud noise cause a medical apparatus carrying out a medical procedure.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device for protection of a subject from noise during a medical procedure, the device comprising:
- an input unit (21) configured to obtain image data of the subject acquired while entering or within a medical apparatus just before and/or at the beginning of and/or during a medical procedure using the medical apparatus (30);
- a processing unit (22) configured to detect from the obtained image data if the subject is wearing ear protection; and
- an output unit (23) configured to output, if it is detected that the subject does not wear ear protection, a notification that the subject does not wear ear protection and/or a control signal configured to prevent the medical apparatus from starting the medical procedure or to modify the medical procedure.

2. Device according to claim 1,
wherein the processing unit (22) is configured to perform image processing of the obtained image data to detect if the subject is wearing ear protection.

3. Device according to claim 1 or 2,
wherein the processing unit (22) is configured to detect if the subject is wearing earmuffs, headphones or ear plugs, in particular by recognizing a pattern or form of earmuffs, headphones or ear plugs in one or more images of the obtained image data.

4. Device according to any one of the preceding claims,
wherein the processing unit (22) is configured to perform the detection and/or the output unit (23) is configured to perform the output of the notification and/or control signal only if the medical apparatus is expected to generate noise above a noise threshold when performing the medical procedure and/or if the medical procedure is modified during its operation so that the medical apparatus is expected to generate noise above the noise threshold.

5. Device according to any one of the preceding claims,
wherein the output unit (23) is configured to output, if it is detected that the subject does wear ear protection and the medical apparatus is expected to generate noise below a noise threshold when performing the medical procedure, a notification that the subject may remove the ear protection.

6. Device according to any claim 4 or 5,
wherein the noise threshold is predetermined or settable by a user, in particular individual for the subject or the type of subject.

7. Device according to any one of the preceding claims,
wherein the input unit (21) is configured to obtain one or more images from a camera that is configured to monitor the medical apparatus, in particular the area of examination and/or the entrance to the area of examination.

8. Device according to any one of the preceding claims,
wherein the medical apparatus is a medical imaging apparatus, in particular a magnetic resonance imaging, MRI, apparatus, configured to acquire image data of the subject in a medical imaging procedure, and
wherein the input unit (21) is configured to obtain image data from the medical imaging apparatus.

9. Device according to claim 8,
wherein the output unit (23) is configured to output a control signal configured to control the medical imaging apparatus to perform an initial image acquisition to acquire image data enabling the detection if the subject is wearing ear protection.

10. Device according to claim 8 or 9,
wherein the output unit (23) is configured to output a control signal configured to control the medical imaging apparatus to perform a silent image acquisition generating noise below a noise threshold, in particular to perform a zero echo time scan if the medical imaging apparatus is an MRI apparatus.

11. System for protection of a subject from noise during a medical procedure, the subject comprising:
- an image acquisition unit (10, 30) configured to acquire image data of the subject while entering or within a medical apparatus just before and/or at the beginning of and/or during a medical procedure using the medical apparatus (30); and
- a device (20) according to any one of the preceding claims for protection of a subject from noise during a medical procedure based on the acquired image data.

12. System according to claim 11,
wherein the image acquisition unit comprises
- a camera (10) configured to monitor the medical apparatus, in particular the area of examination and/or the entrance to the area of examination, and/or
- a medical imaging apparatus (30), in particular a magnetic resonance imaging, MRI, apparatus, configured to acquire image data of the subject in a medical imaging procedure.

13. System according to claim 11 or 12,
further comprising a medical apparatus (30) configured to perform a medical procedure.

14. Method for protection of a subject from noise during a medical procedure, the method comprising:
- obtaining image data of the subject acquired while entering or within a medical apparatus just before and/or at the beginning of and/or during a medical procedure using the medical apparatus;
- detecting from the obtained image data if the subject is wearing ear protection; and
- outputting, if it is detected that the subject does not wear ear protection, a notification that the subject does not wear ear protection and/or a control signal configured to prevent the medical apparatus from starting the medical procedure or to modify the medical procedure.

15. Computer program comprising program code means for causing a computer to carry out the steps of the method as claimed in claim 14 when said computer program is carried out on the computer.
